# EUROPEAN PATENT APPLICATION

(11) **EP 0 751 137 A1**
(43) Date of publication of application: **02.01.1997**
(21) Application number: 96110220.9
(22) Date of filing: 25.06.1996
(51) Int. Cl.: C07D 307/91, A61K 31/34, C07D 307/93

(54) **Oxaindene derivatives and a process for preparing them as well as medicaments containing them and the use of them**

(30) Priority: 27.06.1995 HU 9501887; 27.06.1995 HU 9501888
(71) Applicant: EGIS GYOGYSZERGYAR RT., H-1106 Budapest (HU)
(72) Inventor: Szántay, Csaba, Dr., 1113 Budapest (HU); Novák, Lajos, Dr., 1126 Budapest (HU); Kovács, Péter, 1165 Budapest (HU); Gado, Klára, Dr., 1118 Budapest (HU); Gigler, Gábor, 1067 Budapest (HU); Takács born Julianna Bitai, Gáborné, Dr., 1165 Budapest (HU); Egyed, András, Dr., 1145 Budapest (HU); Bozsing, Dániel, Dr., 1141 Budapest (HU); Pirok, György, 1204 Budapest (HU); Szemerédi, Katalin, Dr., 2011 Budakalász (HU); Csörgö, Margit, Dr., 1174 Budapest (HU); Drabant, Sándor, Dr., 1171 Budapest (HU); Blasko, Gábor, Dr., 1113 Budapest (HU); Simig, Gyula, Dr., 1126 Budapest (HU); Kovács, Gábor, Dr., 1028 Budapest (HU)
(74) Representative: Beszédes, Stephan G., Dr.

(57) **Abstract**

A subject-matter of the invention are oxaindene derivatives of general formula 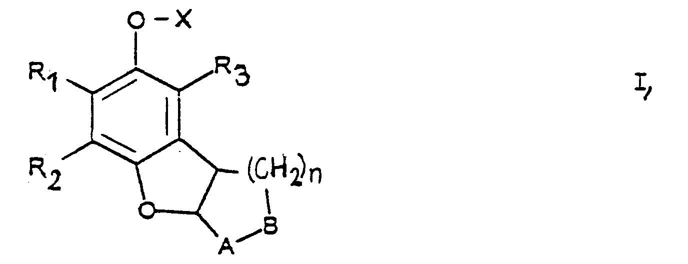 wherein
- R₁: represents a C₁₋₄-alkyl, C₁₋₄-alkoxy or C₃₋₆-cycloalkyl group,
- R₂: stands for hydrogen or a C₁₋₄-alkyl or C₃₋₆-cycloalkyl group,
- R₃: represents hydrogen or a C₁₋₄-alkyl, C₁₋₄-alkoxy or benzyloxy group,
- X: means hydrogen, an aliphatic C₁₋₄-acyl or benzoyl or naphthoyl group, optionally substituted by 1 or more nitro or C₁₋₄-alkoxy group(s) or a group of formula 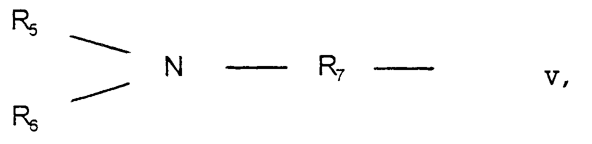 in which latter
- R₇: stands for a C₁₋₄-alkylene group
and
- R₅ and R₆: each represents a C₁₋₄-alkyl group
or
- R₅ and R₆: together with the nitrogen atom to which they are attached form a piperidyl or morpholinyl group,
- A and B: together form an ethylene or a vinylene group
and
- n: is 1, 2 or 3,
and their stereoisomers and their mixtures as well as acid addition salts of these compounds.

Moreover there are provided for a process for preparing these compounds, medicaments containing them and the use of these compounds.

They have a strong 5-lipoxygenase enzyme inhibiting activity.

## Description

The invention is concerned with new oxaindene derivatives including their stereoisomers and their mixtures and salts with useful pharmaceutical activities, particularly against diseases of allergy, diseases of the joints, asthma, diseases of the gastro-intestinal tract including ulcers and skin disorders, and a process for preparing them as well as medicaments containing these compounds and the use of them.

Tricyclic compounds of formula 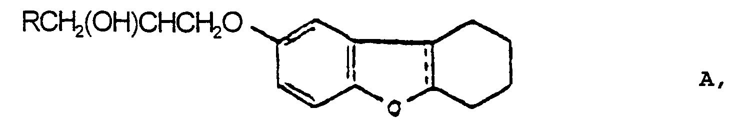 wherein
- R: stands for Me₂CHNH, Me₃CNH, Et₂N, Pr₂N, piperidino, morpholino or 1-imidazolyl, BuNH, cyclohexylamino or 2,6-dimethylpiperidino,
are provided for in Soviet patent application No. 869,278. The compounds have β-adrenoblocking, hypotensive, spasmolytic and neuro-depressive activities and some of them also broncholytic activity.

Benzofurane derivatives fused with a cyclohexene and a cyclopentane ring have been described in J. Org. Chem. 1978, 43 (14), 2 752 to 2 757 and in J. Chem. Soc., Perkin Trans. 1, 1981 (10), 2 760 to 2 766, respectively. No effect is attributed to these compounds.

In published European patent application No. 84,856 tricyclic compounds of formula 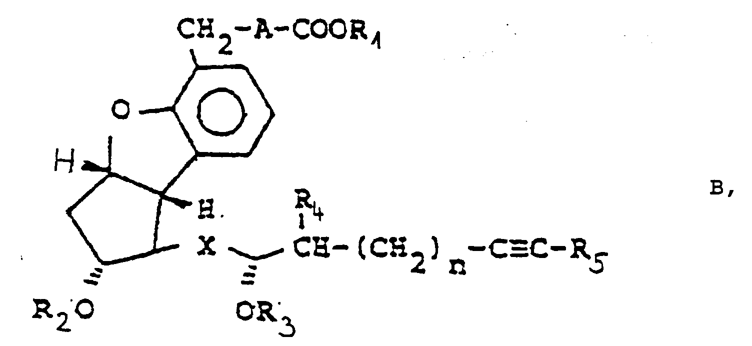 wherein
- R₁: is a pharmaceutically acceptable cation, hydrogen or n-alkyl of 1 to 12 carbon atoms,
- R₂: is hydrogen, acyl of 2 to 10 carbon atoms or aroyl of 7 to 13 carbon atoms,
- R₃: is hydrogen, acyl of 2 to 10 carbon atoms or aroyl of 7 to 13 carbon atoms,
- R₄: is hydrogen, methyl or ethyl,
- R₅: is n-alkyl of 1 to 5 carbon atoms,
- n: is an integer of 0 to 4,
- A: is -CH₂-CH₂- or trans―CH-CH-
and
- X: is -CH₂-CH₂- or trans-CH-CH-,
have been described. Ulcus inhibiting and hypotensive activities are attributed to these compounds.

The problem underlying to the invention is to create novel tricyclic compounds having valuable pharmacological properties, particularly against diseases of allergy, diseases of the joints, asthma, diseases of the gastro-intestinal tract including ulcers and skin disorders, a process for preparing them as well as medicaments containing these compounds and the use of them.

Surprisingly this has been solved by the invention.

Thus a subject-matter of the invention are oxaindene derivatives of general formula 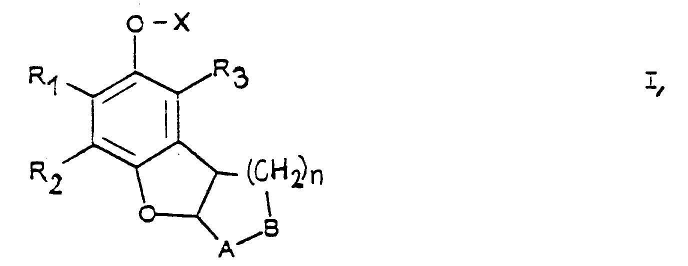 wherein
- R₁: represents a C₁₋₄-alkyl, C₁₋₄-alkoxy or C₃₋₆-cycloalkyl group,
- R₂: stands for hydrogen or a C₁₋₄-alkyl or C₃₋₆-cycloalkyl group,
- R₃: represents hydrogen or a C₁₋₄-alkyl, C₁₋₄-alkoxy or benzyloxy group,
- X: means hydrogen, an aliphatic C₁₋₄-acyl or benzoyl or naphthoyl group, optionally substituted by 1 or more nitro or C₁₋₄-alkoxy group(s) or a group of formula 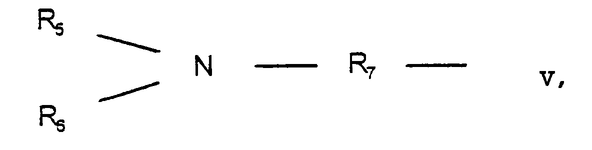 in which latter
- R₇: stands for a C₁₋₄-alkylene group
and
- R₅ and R₆: each represents a C₁₋₄-alkyl group
or
- R₅ and R₆: together with the nitrogen atom to which they are attached form a piperidyl or morpholinyl group,
- A and B: together form an ethylene or a vinylene group
and
- n: is 1, 2 or 3,
and their stereoisomers and their mixtures as well as acid addition salts of these compounds.

The term "alkyl groups" used throughout the text means straight or branched ones, such as methyl, ethyl, n-propyl, isopropyl and n-butyl groups from which the preferred ones are methyl groups. Examples for "cycloalkyl groups", i.e., as above defined, cyclic groups containing 3 to 6 carbon atoms, are cyclopropyl, cyclobutyl, cyclopentyl and cyclohexyl groups. Examples for "alkoxy groups" include methoxy, ethoxy, propoxy and isopropoxy groups, from which the preferred ones are methoxy groups. Examples for aliphatic acyl groups derivable from aliphatic carboxylic acids are acetyl, propionyl and butyryl groups. An example for a substituted benzoyl group is a 4-(nitro)-benzoyl group.

Preferably the alkyl group(s) which may be represented by R₁ , R₂ , R₃ , R₅ and/or R₆ is/are such having from 1 to 3, particularly 1 or 2, above all 1, carbon atom(s).

It is also preferred that the alkoxy group(s) which may be represented by R₁ and/or R₃ and/or by which the benzoyl or naphthoyl group which may be represented by X may be substituted is/are such having from 1 to 3, particularly 1 or 2, above all 1, carbon atom(s).

Furthermore it is preferred that the cycloalkyl group(s) which may be represented by R₁ and/or R₂ is/are such having 5 or 6, particularly 6, carbon atoms.

Moreover it is preferred that the alkylene group which may be represented by R₇ is such having from 1 to 3, particularly 2 or 3, above all 2, carbon atom(s).

It is also preferred that the aliphatic acyl group which may be represented by X is such having from 1 to 3, particularly 2 or 3, above all 2, carbon atom(s).

In a preferred group of the oxaindene derivatives according to the invention
- R₁ and R₃: independently represent hydrogen or C₁₋₄-alkyl or C₁₋₄-alkoxy groups,
- R₂: stands for hydrogen or a C₁₋₄-alkyl group,
- X: represents hydrogen,
- A and B: together form a vinylene group
and
- n: is 1 or 2.

In another preferred group of the oxaindene derivatives according to the invention
- R₁ and R₃: independently represent hydrogen or C₁₋₄-alkyl or C₁₋₄-alkoxy groups,
- R₂: stands for hydrogen or a C₁₋₄-alkyl group,
- X: represents hydrogen,
- A and B: together form an ethylene group
and
- n: is 1 or 3.

Particularly preferred representatives of the oxaindene derivatives according to the invention are the following ones:
cis-4,6,7-tri-(methyl)-2,3,3a,8a-tetra-(hydro)-1H-8-oxacyclopenta[a]indene-5-ol, 1,2,4-tri-(methyl)-4b,6,7,8,9,9a-hexa-(hydro)-5H-10-oxabenzo[a]azulene-3-ol and cis-4,6,7-tri-(methyl)-3a,8a-di-(hydro)-3H-8-oxacyclopenta-[a]indene-5-ol.

The oxaindene derivatives of general formula I, wherein X represents a group of formula V, can form acid addition salts with acids. Suitably they are salts with pharmaceutically useful acids. They can be formed with inorganic acids, e.g. hydrohalides, such as hydrochloric acid or hydrobromic acid, sulfuric acid, phosphoric acid or perhaloacids, such as perchloric acid, organic carboxylic acids, e.g. fumaric, acetic, propionic, glycolic, maleic, hydroxymaleic, ascorbinic, citric, malic, salicylic, lactic, cinnamic, benzoic, phenylacetic, p-aminobenzoic, p-hydroxybenzoic or p-aminosalicylic acid, alkylsulfonic acids, e.g. methanesulfonic or ethanesulfonic acid, or arylsulfonic acids, e.g. p-toluenesulfonic, p-bromophenylsulfonic, naphthylsulfonic or sulfanilic acid.

According to a further aspect of the present invention there is provided for a process for preparing the oxaindene derivatives according to the invention, which is characterized by that
a) for preparing oxaindene derivatives of general formula I, in which A and B together form a vinylene group and R₁ , R₂ , R₃ , X and n are as above defined, reacting hydroquinone derivatives of general formula 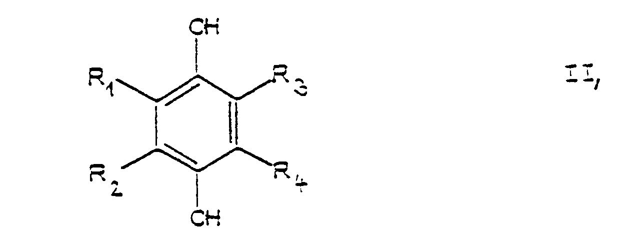 wherein R₁ , R₂ and R₃ are as above defined and R₄ represents hydrogen or a C₁₋₄-alkoxy group, with cycloalkene derivatives of general formula 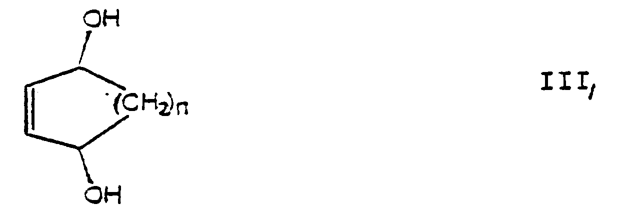 wherein n is as above defined, in solvents in the presence of catalytic amounts of strong acids, and, in a manner known per se, optionally subjecting thus obtained oxaindene derivatives of general formula I, wherein X represents hydrogen, to acylation or dialkylaminoalkylation
   or
b) for preparing oxaindene derivatives of general formula I, in which A and B together form an ethylene group,
   b₁) in a manner known per se subjecting oxaindene derivatives of general formula I, wherein A and B together form a vinylene group, X stands for hydrogen and R₁ , R₂ , R₃ and n are as above defined, to catalytic hydrogenation,
      or
   b₂) reacting hydroquinone derivatives of general formula II, wherein R₁ , R₂ and R₃ are as above defined and R₄ represents hydrogen, with cycloalkadiene derivatives of general formula 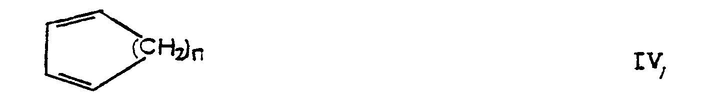 wherein n is as above defined, in solvents in the presence of catalytic amounts of strong acids,
   and, in a manner known per se, optionally subjecting thus obtained oxaindene derivatives of general formula I, wherein X represents hydrogen, to acylation or dialkylaminoalkylation,
and, in a manner known per se, if desired, converting oxaindene derivatives of general formula I thus obtained into acid addition salts thereof or, if desired, liberating free oxaindene derivative bases of general formula I from salts thereof and, if desired, converting the free oxaindene derivative bases into other acid addition salts and/or, if desired, separating the stereoisomers.

In variant a) of the process according to the invention preferably for the reaction of the hydroquinone derivatives of general formula II with the cycloalkene derivatives of general formula III [variant a) of the process according to the invention] as solvent(s) [an] aprotic, advantageously anhydrous, solvent(s), particularly 1 or more aromatic hydrocarbon(s), such as benzene, toluene and/or [a] xylene(s), is/are used further examples of usable aprotic solvents being 1 or more halogenated hydrocarbon(s), e.g. methylene chloride, chloroform and/or 1,2-di-(chloro)-ethane. It is also preferred that for the reaction of the hydroquinone derivatives of general formula II with the cycloalkene derivatives of general formula III [variant a) of the process according to the invention] or with the cycloalkadiene derivatives of general formula IV [variant b₂) of the process according to the invention] as strong acid p-toluenesulfonic acid, camphor-10-sulfonic acid or benzenesulfonic acid is used camphor-10-sulfonic acid being most preferred. Suitably the reaction of the hydroquinone derivatives of general formula II with the cycloalkene derivatives of general formula III [variant a) of the process according to the invention] is carried out at elevated temperatures, particularly of from 60 to 110°C. The thus obtained oxaindene derivatives of general formula I according to the invention can be isolated e.g. by cooling the reaction mixture to room temperature, shaking it with some water, separating the phases, extracting the aqueous phase with an organic solvent, e.g. dichloromethane, washing and drying the combined organic phases and distilling off the solvent. The thus obtained oxaindene derivatives of general formula I are optionally purified by crystallization or column chromatography.

Preferably as solvent(s) for the catalytic hydrogenation of the oxaindene derivatives of general formula I, wherein A and B together form a vinylene group, X stands for hydrogen and R₁ , R₂ , R₃ and n are as above defined, according to variant b₁) of the process according to the invention 1 or more polar solvent(s), particularly 1 or more C₁₋₄-alcohols, above all methanol, is/are used. As catalyst for the catalytic hydrogenation of the oxaindene derivatives of general formula I, wherein A and B together form a vinylene group, X stands for hydrogen and R₁ , R₂ , R₃ and n are as above defined, of variant b₁) of the process according to the invention preferably palladium, e.g. palladium applied onto charcoal, is used. This catalytic hydrogenation can be performed at temperatures of from 0 to 30°C, under atmospheric pressure or under a slightly elevated pressure. One works preferably at room temperature and under atmospheric pressure. The thus obtained oxaindene derivatives of general formula I, wherein X stands for hydrogen, can be separated from the reaction mixture by filtering off the catalyst, evaporating the filtrate and optionally purifying the residue by recrystallization or column chromatography.

Preferably the reaction of the hydroquinone derivatives of general formula II with the cycloalkadiene derivatives of general formula IV [variant b₂)of the process according to the invention] is carried out in 1 or more anhydrous aprotic solvent(s), such as benzene, toluene and/or [a] xylene(s). It is also preferred to carry out the reaction of the hydroquinone derivatives of general formula II with the cycloalkadiene derivatives of general formula IV [variant b₂) of the process according to the invention] at elevated temperatures, particularly of from 30 to 90°C. The thus obtained oxaindene derivatives of general formula I, wherein X stands for hydrogen, can be isolated from the reaction mixture, e.g. by cooling the latter to room temperature, washing, drying and evaporating it. The residue may optionally be purified by recrystallization or column chromatography.

The optional acylation of thus obtained oxaindene derivatives of general formula I, wherein X represents hydrogen, can be carried out with organic carboxylic acids containing the acyl group to be introduced, or with functional derivatives thereof, e.g. acid halides or acid anhydrides. As acid halides preferably acid chlorides are used.

If carboxylic acids are used as acylating agents, the reaction is preferably carried out in aprotic dipolar solvents, such as dimethylformamide, in the presence of condensing agents. As condensing agents e.g. compounds of the carbodiimide type, preferably dicyclohexylcarbodiimide, may be used.

The acylation carried out with acid anhydrides can be performed in apolar aprotic solvents, such as ether, dioxane, benzene, chloroform and/or dichloromethane. Suitably the reaction is carried out in the presence of acid binding agents, e.g. alkali carbonates, sodium hydride or organic bases. Certain organic bases, e.g. pyridine, can serve both as solvents and as acid binding agents.

The acylation carried out with acid halides, preferably acid chlorides, can be performed in inert organic solvents. As reaction medium aliphatic ethers, e.g. diethyl ether and/or tetrahydrofurane, and/or, optionally halogenated, aliphatic hydrocarbons, e.g. dichloromethane, can be used, but the acid binding agent, e.g. pyridine, may serve as reaction medium as well.

The reaction temperature of the acylation may be varied between wide ranges, but it is preferable to carry out the reaction at room temperature. It is preferably performed in the presence of an acid binding agent. For this purpose organic tertiary amine bases, e.g. triethylamine, pyridine or N-(methyl)-morpholine, can be used. In certain cases the organic bases may serve both as solvents and acid binding agents.

The optional dialkylaminoalkylation of the oxaindene derivatives of general formula I, wherein X stands for a hydrogen atom, can be carried out with N,N-di-(alkyl)-aminoalkylhalides of general formula 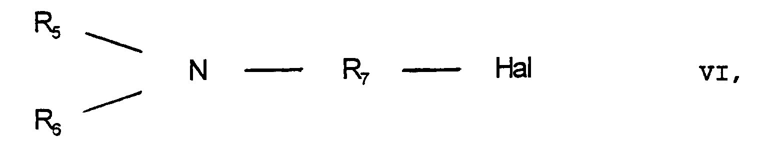 wherein
- R₅ , R₆ and R₇: are as above defined
and
- Hal: stands for halogen.
This dialkylaminoalkylation is preferably performed in the presence of a base, e.g. an alkali hydroxide, alkali carbonate, alkali alkoxide, sodium hydride or an organic base.

The starting hydroquinone derivatives of general formula II, cycloalkene derivatives of general formula III and cycloalkadiene derivatives of general formula IV used in the process according to the invention for preparing the oxaindene derivatives of general formula I are known. They are available from the commerce, or can be prepared as described in the following publications: W. Baker: Journal of Chemical Society 1941, 662; L. N. Owen et al.: ibid. 1952, 4 035, E. Knoevenagel et al.: Berichte, 34, 3 993 [1901], J. E. Backvall et al.: Journal of Organic Chemistry 49, 4 619 [1984].

The oxaindene derivatives according to the invention are strong inhibitors of the 5-lipoxygenase (5-LO) enzyme. They are more effective by one or two order(s) of magnitude than AA-861, the well-known selective 5-LO inhibitor. As already mentioned, these 5-lipoxygenase inhibitors effective both in vitro and in vivo have useful pharmaceutical activities, particularly against diseases of allergy, e.g. allergic rhinitis, diseases of the joints, e.g. rheumatoid arthritis, diseases of the gastro-intestinal tract, e.g. ulcerous colitis, asthma and skin disorders, e.g. psoriasis.

Hence by the invention also there are provided for medicaments, which are characterized by that they contain as active principle(s) at least one oxaindene derivative according to the invention, suitably in admixture with at least one inert solid and/or liquid pharmaceutical carrier and/or diluent and/or further auxiliary excipient.

The efficacy of the oxaindene derivatives according to the invention is demonstrated by the following experiments:
A) Measurement of 5-LO enzyme activity in cell free system prepared from human leukocytes
   The enzyme activity was measured according to the modified method of Ochi et al. [K. Ochi, T. Yoshimoto, S. Yamamoto, K. Taniguchi, T. Miyamoto: Arachidonate 5-lipoxygenase of guinea pig peritoneal polymorphonuclear leukocytes, J. Biol. Chem. 258, 5754-5758 (1983)]. The principle of the method is as follows: in in vitro systems, during the reaction catalysed by 5-LO 5-hydroperoxy-6,8,11,14-eicosatetraenoic acid (5-HPETE) is formed from arachidonic acid, then it is transformed to 5-hydroxy-6,8,11-14-eicosatetraenoic acid (5-HETE). Both 5-HPETE and 5-HETE have absorption maximums at 236 nm, therefore it is possible to determine the activity of 5-LO enzyme by measuring the change of absoprtion at 236 nm. 5-LO was prepared from human polymorphonuclear leukocytes by a simple purification procedure. The cells were washed and lysed by a sonic disintegrator. The lysate was centrifuged and the enzyme in the cytosol was partially purified by ammonium sulphate fractionation (the enzyme precipitates at 50% saturation). The reaction mixture (50 mM tris-HCL pH=7.5, 2 mM CaCl₂, 0.1 mM ATP [adenosin-5'-triphosphate], 0.02 mM arachidonic acid) was incubated at 37°C for 5 minutes. The reaction was started with the addition of arachidonic acid and stopped by the addition of 10 mM of citric acid. The thus-obtained 5-PHETE and 5-HETE were extracted with a mixture of diethyl ether and hexane in a ratio by volume of 6 : 4 and the absorption of the organic phase was measured by spectrophotometry at 238 nm.
B) Determination of 5-LO enzyme activity of human leukocytes by measuring their leukotriene B₄ [LTB₄] production
   The method of Mita et al. was applied [H. Mita, Y. Yui and T. Shida: Effect of AA-861, a 5-lipoxygenase inhibitor on leukotriene synthesis in human polymorphonuclear leukocytes and on cyclooxygenase and 12-lipoxygenase activities in human platelets, Allergy 41, 493-498, (1986)]. The principle of the experiment is as follows: the calcium ionophore A-23187 induces the synthesis and the release of LTB₄ by human polymorphonuclear leukocytes. The amount of LTB₄ released into the incubation medium reflects the 5-LO enzyme activity of the intact cells. The incubation medium was Hank's balanced medium (HBBS) supplemented with 17 mM of Tris-HCl (pH 7.2), 1.22 mM of CaCl₂ , 1 mM of MgCl₂ , 0.001 mM of A-23187 and 10⁴ PMN leukocyte per ml reaction mixture. The reaction was started with the addition of ionophore, then incubated at 37°C for 15 minutes. The reaction was stopped by adding 5 mM of EDTA by chilling the samples in an ice bath. The samples were centrifuged and the amount of LTB₄ in the supernatant was determined by radioimmunoassay (RIA).
C) Ear oedema induced by arachidonic acid in mice
   Inflammation of ear was induced by arachidonic acid using a modified version of the procedure described by Young et al. [Young, J.M., Spires, D.A., Bedord, C.J., Wagner, B., Ballaron, S.J., De Young, L.M.: The mouse ear inflammatory response to topical arachidonic acid, J. Invest. Dermatol. 82, 367-371 (1984)]. The experiments were carried out in female mice from the NMRI strain weighing 25 to 30 g (8 animals/group). Drugs or vehicle were applied topically in acetone or ethanol by an automatic pipette in 10 µl volumes to both the inner and outer surfaces of the ear. 30 minutes after drug or vehicle administration both sides of the left ear were brushed with 3 mg of arachidonic acid in 20 µl of acetone and both sides of the right ear were treated with 20 µl of acetone only. 60 minutes later the animals were killed by ether, then a disk of 7 mm diameter was cut off from both ears. The mass of each disk was measured by an analytical scale and the mean of the difference of the left and right ears was calculated for every group. ID₅₀ values were determined by blotting the log-dose versus precentage response as compared to vehicle-treated animals.
D) PAF induced anaphylactic shock test in mice
   A modification of the method of Young et al. [Young, J.M., Maloney, P.J., Jubb, S.N. and Clark, J.S.: Pharmacological investigation of the mechanisms of platelet-activating factor induced mortality in the mouse, Prostaglandins 30, 545-551 (1985)] was used. The experiments were carried out in male NMRI mice weighing 20-25 g (10 animals/group). The test compounds were suspended in an aqueous 0.4 % by weight methyl cellulose solution and administered intraperitoneally in a volume of 10 ml/kg. The mice of the control group received only vehicle. 30 minutes later the animals received intravenous PAF in a dose of 100 µg/kg (5 ml/kg). Mortality was determined one hour after PAF administration. Every experiment was repeated three times (n=30). The ratio of mortality of the treated animals was compared to that of the control mice. ID₅₀ values were calculated by linear regression analysis.
   The results are shown in the following Table. As reference compounds 1-(phenyl)-pyrazolidin-3-one {phenidone}, 2-[12'-(hydroxy)-dodeca-5',10'-di-inyl]-3,5,6-tri-[methyl]-p-benzoquinone {AA-861} and nordihydroguaiaretic acid {1,4-bis-[3,4-di-(hydroxy)-phenyl]-2,3-di-[methyl] -butane} 〈NDGA〉 were applied.

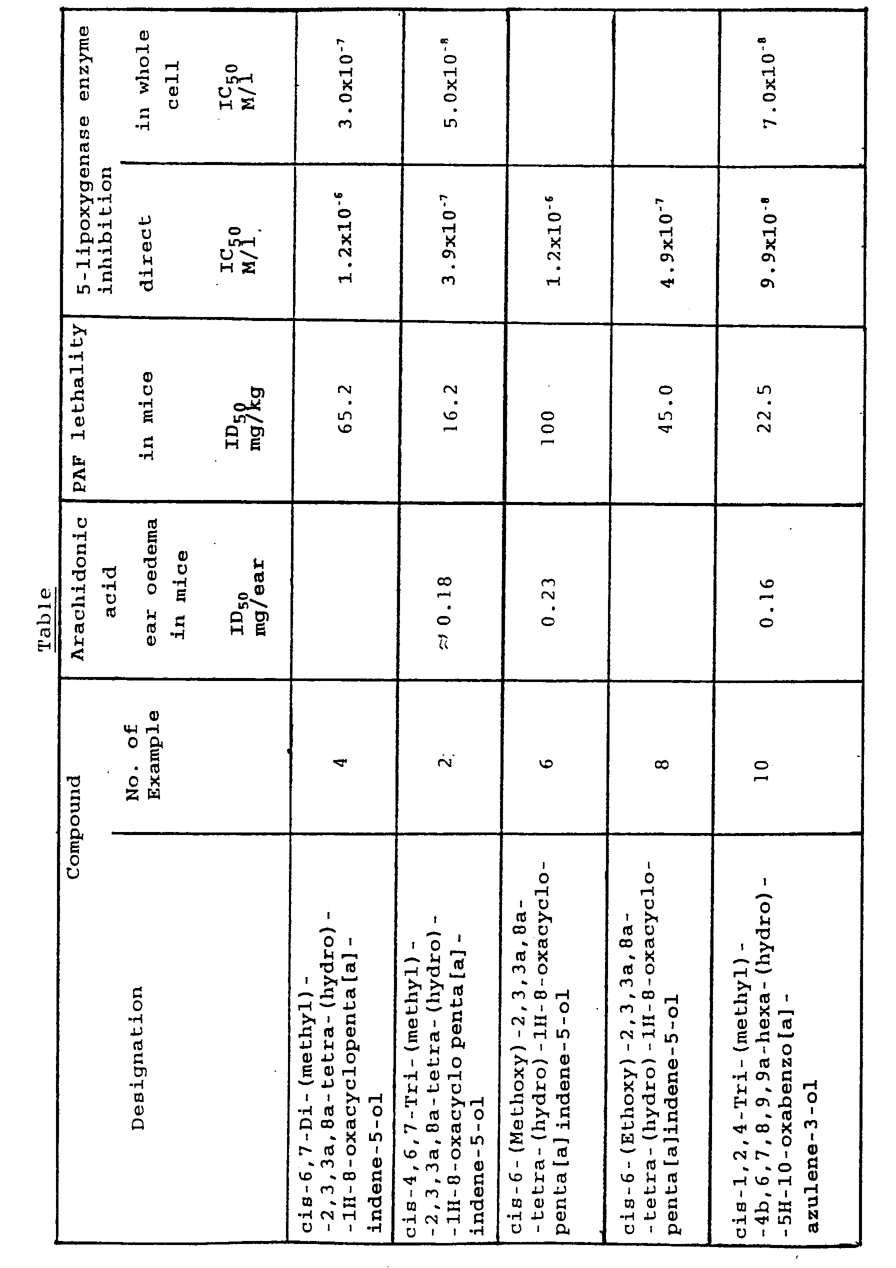

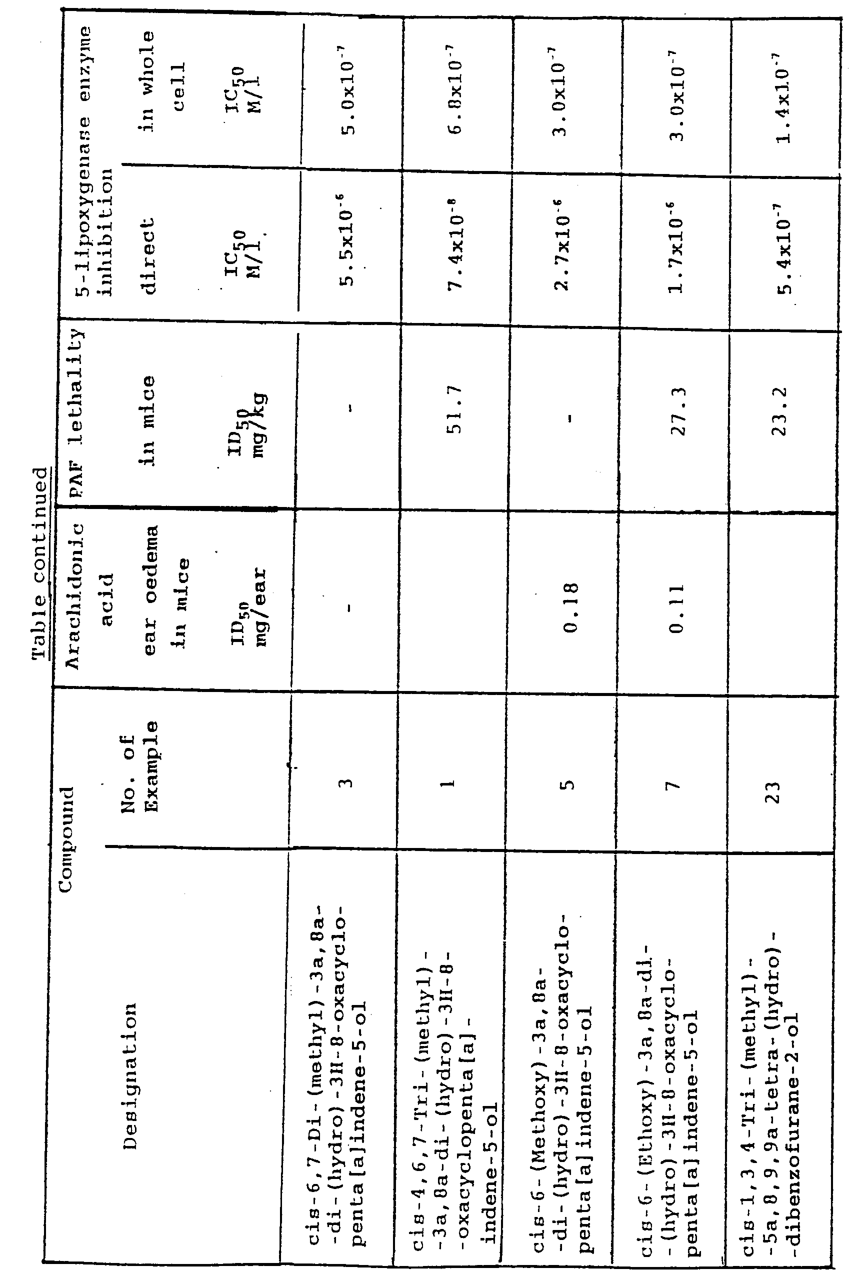

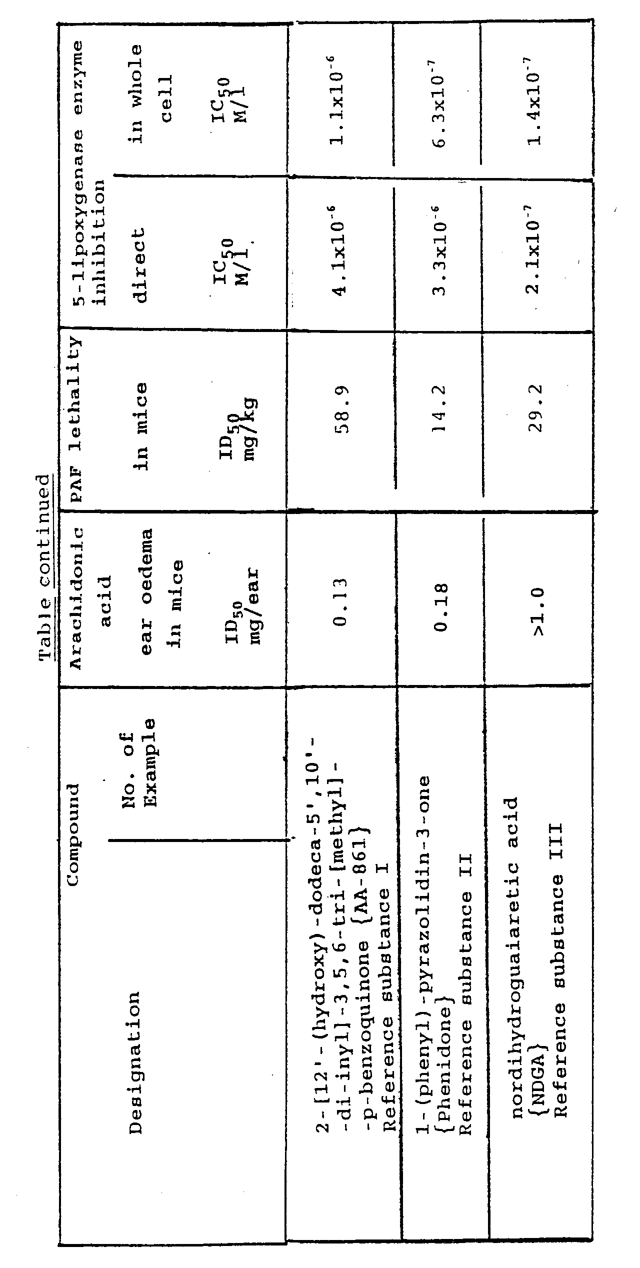

As it can be seen from the above Table, the activity of the tested oxaindene derivatives according to the invention is equal to superior to those of the selective 5-lipoxygenase enzym inhibitor 2-[12'-(hydroxy)-dodeca-5',10'-di-inyl]-3,5,6-tri-[methyl]-p-benzoquinone {AA-861} 〈reference substance I〉 and of the dual inhibitors 1-(phenyl)-pyrazolidin-3-one {phenidone} 〈reference substance II〉 and nordihydroguaiaretic acid {NDGA} 〈reference substance III〉.

The medicaments of the present invention can be prepared by methods known per se by admixing the active principle(s) with the inert, non-toxic, solid and/or liquid pharmaceutical carrier(s) and/or diluent(s) and/or further auxiliary excipient(s) and bringing the mixture to galenic form.

Preferably the medicaments according to the invention are in form of tablets or dragées, but advantageously they can be in the form of solutions or suspensions, too. The daily dose is generally 1 to 800 mg, preferably 10 to 500 mg.

According to a further aspect of the present invention there is provided for the use of the oxaindene derivatives according to the invention for the preparation of medicaments, particularly such having activities against diseases of allergy, diseases of the joints, asthma, diseases of the gastro-intestinal tract including ulcers and skin disorders.

The medicaments according to the invention can be used by administering to the patient an effective amount of the oxaindene derivatives according to the invention.

The invention is further illustrated by the following Examples the ratios of the components of the mixed solvents in all Examples being by volume.

### Example 1

### cis-4,6,7-Tri-(methyl)-3a,8a-di-(hydro)-3H-8-oxacyclopenta[a]indene-5-ol (I: R₁=R₂=R₃=Me, X=H, A+B=vinylene, n=1)

To a suspension of 35.0 g (0.23 mole) of trimethylhydroquinone (II: R₁=R₂=R₃=Me, R₄=H) and 25.0 g (0.25 mole) of cis-cyclopent-4-ene-1.3-diol (III: n=1) in 300 ml of anhydrous toluene 2.0 g of D-camphor-10-sulfonic acid are added, and the mixture is stirred at a temperature of 70 ^{o}C for 40 hours. Then it is cooled, the separated crystals are filtered off and recrystallized from ethanol. Thus 25 g (50.3 %) of white needle crystals are obtained.
M.p.: 14O ^{o}C
¹H-NMR (CDCl₃): 2.10 (3H, s, CH₃). 2.12 (3H, s, CH₃), 2.17 (3H, s, CH₃), 2.52 and 2.92 (2H, m, CH₂), 4.08 (1H, m, CH), 4.16 (1H, s, OH), 5.79 (1H, m, CH), 5.91 (1H, m, CH=), 6.00 (1H, m, CH=).

### Example 2

### cis-4,6,7-Tri-(methyl)-2,3,3a,8a-tetra-(hydro)-1H-8-oxacyclopenta[a]indene-5-ol (I: R₁=R₂=R₃=Me, X=H, A+B=ethylene, n=1)

a) 4.0 g of the product prepared according to Example 1 is dissolved in 300 ml of anhydrous methanol and hydrogenated using as catalyst 0.2 g of 10% by weight of palladium on charcoal. When the theoretical amount of hydrogen has been taken up (450 ml, 45 minutes) the catalyst is filtered off, the filtrate is evaporated and the residue is recrystallized from ethanol. Thus 3.5 g (86 %) of white crystalline product are obtained.
   M.p.: 141°C.
   ¹H-NMR(CDCl₃): 1.5-1.8 (5H, m, 5H of the 3CH₂ group), 1.8-2.0 (1H, m, H of the CH₂ group), 2.09 (3H, s CH₃), 2.12 (3H, s, CH₃), 2.16 (3H, s, CH₃), 3,76 (1H, m-t, J=7Hz, CH-O).
b) To a solution of 15.2 g (0,1 mole) of trimethylhydroquinone (II: R₁=R₂=R₃=Me and R₄=H) and 7.26 g (0.11 mole) of cyclopenta-1,3-diene (IV: n=1) in 225 ml of anhydrous toluene and 75 ml of anhydrous ether 3.8 g (22 mmoles) of p-toluenesulfonic acid are added, and the mixture is heated in an oil bath of 70 ^{o}C for 14 hours. Then it is cooled, 200 ml of ether are added to it, washed with water, dried, the solvent is distilled off and the residue is recrystallized from methanol. Thus 13.2 g (60.5 %) of white crystalline product are obtained.
   M.p.: 141 ^{o}C.

### Example 3

### cis-6,7-Di-(methyl)-3a,8a-di-(hydro)-3H-8-oxacyclopenta[a]indene-5-ol (I: R₁=R₂=Me, R₃=X=H, A+B=vinylene, n=1)

To a suspension of 11.5 g (83 mmoles) of 2,3-di-(methyl)-hydroquinone (II: R₁=R₂=Me, R₃=R₄=H) and 8.34 g (83 mmoles) of cis-cyclopent-4-ene-1.3-diol (III: n=1) in 90 ml of anhydrous toluene 0.9 g of D-camphor-10-sulfonic acid is added, and the mixture is stirred at a temperature of 70 ^{o}C for 40 hours. Then it is cooled, diluted with 400 ml of ethyl acetate and washed with 200 ml of water. The aqueous phase is extracted with 100 ml of ethyl acetate, the organic phases are combined and washed with water and saturated sodium chloride solution, dried and the solvent is distilled off. The residue is purified by column chromatography (Kieselgel G., hexane:acetone 10:1). Thus 5.65 g (34 %) of white needle crystals are obtained.
M.p.: 159 ^{o}C.
¹H-NMR (CDCl₃): 2.11 (6H, s, 2CH₃), 2.52 and 2.88 (2H, m, CH₂), 3.8 (1H, s, OH), 4.0 (1H, m, CH), 5.76 (1H, m, CH-O), 5.84 (1H, m, CH=), 5.98 (1H, m, CH=), 6.48 (1H, s, aromatic pr.).

### Example 4

### cis-6,7-Di-(methyl)-2,3,3a,8a-tetra-(hydro)-1H-8-oxacyclopenta[a]indene-5-ol (I: R₁=R₂=Me, R₃=X=H, A+B=ethylene, n=1)

a) 3.0 g (15 mmoles) of the product prepared according to Example 3 are hydrogenated in 150 ml of ethanol using 0.2 g of 5% by weight of palladium on charcoal catalyst. When the theoretical amount of hydrogen has been taken up (360 ml, 45 minutes) the catalyst is filtered off, the solvent is distilled off, and the residue is crystallized from ethanol. Thus 1.9 g (63 %) of white crystalline product is obtained.
   M.p.: 142 ^{o}C.
   ¹H-NMR (CDCl₃): 1.5 (1H, m, one H of CH₂), 1.6-1.9 (4H, m, 2CH₂), 2.05 (1H, m, one H of CH₂), 3.77 (1H, t+d, J=7 and 1Hz, CH), 4.4 (1H, s, OH), 5.20 (1H, m-t, J=6Hz, CH-O), 6.46 (1H, s, aromatic pr.).
b) 13.8 g (0.11 mole) of 2,3-di-(methyl)-hydroquinone (II: R₁=R₂=Me, R₃=R₄=H) and 7.26 g (0.11 mole) of cyclopenta-1,3-diene (IV: n=1) are dissolved in the mixture of 75 ml of anhydrous ether and 225 ml of anhydrous toluene, 8 g (22 mmole) of p-toluenesulfonic acid are added to the solution, and the reaction mixture is heated in an oil bath of 80 ^{o}C for 16 hours. The mixture is then cooled, diluted with 200 ml of ether, washed with water, dried and the solvent is distilled off. The residue is recrystallized from ethanol. Thus 10.3 g (50.5 %) of white crystalline product are obtained.
   M.p.: 142 ^{o}C.

### Example 5

### cis-6-(Methoxy)-3a,8a-di-(hydro)-3H-8-oxacyclopenta[a]indene-5-ol (I: R₁=MeO, R₂=R₃=X=H, A+B=vinylene, n=1)

To a solution of 8.0 g (47.1 mmoles) of 2,5-di-(methoxy)-hydroquinone (II: R₁=R₄=MeO, R₂=R₃=H) and 5.0 g (51 mmoles) of cis-cyclopent-4-ene-1,3-diol (III: n=1) in 100 ml of anhydrous toluene O.5 g of D-camphor-10-sulfonic acid is added, and the reaction mixture is stirred under argon atmosphere at a temperature of 70 ^{o}C for 6 hours. Then it is cooled, the separated side-product [2,5-di-(methoxy)-1,4-benzoquinone] is filtered off, the filtrate is evaporated, the residue is purified by column chromatography and recrystallized from hexane. Thus 1.3 g (28 %) of white crystalline product is obtained.
M.p.: 116-118 ^{o}C.
¹H-NMR(CDCl₃): 2.52 and 2.86 (2H, m, CH₂), 3.81 (3H, s, OCH₃), 4.00 (1H, m, CH), 5.25 (1H, s, OH), 5.79 (1H, m, CH-O), 5.82 (1H, m, CH=), 6.00 (1H, m, CH=), 6.38 (1H, s, aromatic pr.), 6.72 (1H, m, aromatic pr.).

### Example 6

### cis-6-(Methoxy)-2,3,3a,8a-tetra-(hydro)-1H-8-oxacyclopenta[a]indene-5-ol (I: R₁=MeO, R₂=R₃=X=H, A+B=ethylene, n=1)

a) The solution of 3.6 g (19 mmoles) of the product prepared as specified in Example 5 in 200 ml of methanol is subjected to hydrogenation using as catalyst 1.0 g of 5% by weight of palladium applied on charcoal. When the theoretical amount of hydrogen has been taken up (460 ml, 20 minutes) the catalyst is filtered off, the filtrate is evaporated and the residue is subjected to chromatography on a short column, (Kieselgel 60, hexane-acetone 10:0.3). Thus 0.7 g (19 %) of white crystalline product is obtained.
   M.p.: 100-103 ^{o}C.
   ¹H-NMR (CDCl₃): 1.49 (1H, m, CH₂), 1,6-1,9 (4H, m, 2CH₂), 2.03 (1H, m, CH₂), 3.76 (1H, m-t, J=8Hz, CH), 3.81 (3H, s, OCH₃), 5.16 (1H, s, OH), 5.22 (1H, m-t, O-CH), 6.32 (1H, s, aromatic pr.), 6.68 (1H, s, aromatic pr.).
b) 5.0 g (36 mmoles) of 2-(methoxy)-hydroquinone (II: R₁ = MeO, R₂=R₃=R₄=H) and 7.0 g of cyclopenta-1,3-diene (IV: n = 1) are dissolved in the mixture of 60 ml of anhydrous toluene and 20 ml of ether, 5.0 g of D-camphor-10-sulfonic acid are added to the solution, and it is stirred at a temperature of 40 ^{o}C for 24 hours. Then it is cooled, the separated crystals are filtered off, the solvent is distilled off and the residue is purified by column chromatography. Thus 1.0 g (15 %) of colourless oil is obtained.
   TLC: R_{f}=0,6 (hexane-acetone 10:4)

### Example 7

### cis-6-(Ethoxy)-3a,8a-di-(hydro)-3H-8-oxacyclopenta[a]indene-5-ol (I: R₁=EtO, R₂=R₃=X=H, A+B=vinylene, n=1)

To a mixture of 14.5 g (73 mmoles) of 2.5-di-(ethoxy)-hydroquinone (II: R₁=R₄=EtO, R₂=R₃=H) and 7.9 g (81 mmoles) of cis-cyclopent-4-ene-1,3-diol (III: n=1) in 140 ml of anhydrous toluene 0.7 g of D-camphor-10-sulfonic acid is added, and the mixture is stirred under argon atmosphere at a temperature of 70 ^{o}C for 30 hours. Then it is cooled, the separated 2,5-di-(ethoxy)-1,4-benzoquinone is filtered off, the mother liquor is evaporated, the residue is purified by column chromatography (Kieselgel, hexane:acetone 5:0.2) and recrystallized from hexane. Thus 4.1 g (51.5 %) of colourless crystalline product are obtained.
M.p.: 94-96 ^{o}C
¹H-NMR (CDCl₃): 1.40 (3H, t, J=6Hz, CH₃), 2.52 and 2.86 (2H, m, CH₂), 4.02 (3H, m, CH and CH₂-O), 5.28 (1H, s, OH), 5.79 (1H, m, CH-O), 5.82 (1H, m, CH=), 6.00 (1H, m, CH=), 6.38 (1H, s, aromatic pr.), 6.72 (1H, s, aromatic pr.).

### Example 8

### cis-6-(Ethoxy)-2,3,3a,8a-tetra-(hydro)-1H-8-oxacyclopenta[a]indene-5-ol (I: R₁=EtO, R₂=R₃=X=H, A+B=ethylene, n=1)

A solution of 4.0 g (18.3 mmole) of the product prepared as specified in Example 7 in 200 ml of anhydrous methanol is subjected to hydrogenation using 1.0 g of 5% by weight of palladium on charcoal catalyst. When the theoretical amount of hydrogen has been taken up (440 ml, 20 minutes) the catalyst is filtered off, the solvent is distilled off and the residue is recrystallized from hexane. Thus 2.0 g (50 %) of white crystalline product are obtained.
M.p.: 94-96 ^{o}C.
¹H-NMR (CDCl₃): 1.42 (3H, t, J=6, 5Hz, CH₃), 1.48 (1H, m, CH₂), 1.6-1.9 (4H, m, 2CH₂), 2.04 (1H, m, CH₂), 3.77 (1H, m, J=8Hz and 2.5Hz, CH), 4.03 (2H, q, J=6,5Hz, OCH₂), 5, 22 (1H, m, J=8Hz, O-CH), 5.26 (1H, s, OH), 6.30 (1H, s, aromatic pr.), 6.69 (1H, s, aromatic pr.).

### Example 9

### cis-1,2,4-Tri-(methyl)-4b,6,7,9a-tetra-(hydro)-5H-10-oxabenzo[a]azulene-3-ol (I: R₁=R₂=R₃=Me, X=H, A+B=vinylene, n=3)

To a solution of 10.0 g (66 mmoles) of trimethylhydroquinone (II: R₁=R₂=R₃=Me, R₄=H) and 14.0 g (0.109 mole) of cis-cyclohept-2-ene-1,4-diol (III: n=3) in 150 ml of anhydrous toluene 2,0 g of p-toluenesulfonic acid are added, and the mixture is stirred at a temperature of 75 ^{o}C for 6 hours. Then it is cooled, 100 ml of ether are added to it, washed with water and saturated sodium chloride solution, dried and the solvent is distilled off. The residue is purified by column chromatography (Kieselgel 60, hexane:ether 1:1). Thus 4.8 g (30 %) of white solid substance are obtained.
¹H-NMR (CDCl₃): 1.5-1.9 (6H, m, 3CH₂), 2.02 (6H, s, 2CH₃), 2.08 (3H, s, CH₃), 3.25 (1H, m, CH), 4.18 (1H, s, OH), 5.22 (1H, m, CH-O), 5.4-6.2 (2H, m, CH=CH).

### Example 10

### cis-1,2,4-Tri-(methyl)-4b,6,7,8,9,9a-hexa-(hydro)-5H-10-oxabenzo[a]azulene-3-ol (I: R₁=R₂=R₃=Me, X=H, A+B=ethylene, n=3)

a) 3.0 g (12.3 mmole) of the compound prepared as specified in Example 9 are hydrogenated in 100 ml of methanol using 0.1 g of 10% by weight of palladium on charcoal catalyst. When the calculated amount of hydrogen has been taken up (300 ml, 1 hour) the catalyst is filtered off, the solvent is distilled off in vacuo and the residue is recrystallized from hexane. Thus 1.8 g (59 %) of white crystalline product is obtained.
   M.p.: 112-114 ^{o}C.
   ¹H-NMR(CDCl₃): 1.28-1.95 (9H, m, 9H of 5CH₂), 2.11 (3H, s, CH₃), 2.13 (3H, s, CH₃) 2.15 (3H, s, CH₃), 2.25 (1H, m, one H of CH₂), 3.33 (1H, m, CH), 4.16 (1H, s, OH), 4.79 (1H, m, CH-O).
b) To a suspension of 24.73 g (0.16 mole) of trimethylhydroquinone (III: R₁=R₂=R₃=Me, R₄=H) and 17.0 g (0.18 mole) of cyclohepta-1,3-diene (IV: n=3) in 250 ml of anhydrous toluene 2.0 g of p-toluenesulfonic acid are added, and the mixture is stirred at a temperature of 70 ^{o}C for 16 hours. Then it is cooled, diluted with 250 ml of ether, washed with water, dried, the solvent is distilled off and the residue is recrystallized from a 1 : 1 mixture of hexane and ether. Thus 9.8 g (24.5 %) of white crystalline product are obtained.
   M.p.: 112-114 ^{o}C.

### Example 11

### cis-[4,6,7-Tri-(methyl)-2,3,3a,8a-tetra-(hydro)-1H-8-oxacyclopenta[a]indene-5-yl] benzoate (I: R₁=R₂=R₃=Me, X=benzoyl, A+B=ethylene, n=1)

To a solution of 2.18 g (10 mmoles) of the product prepared according to Example 2 in 25 ml of anhydrous pyridine 1.6 g (11.4 mmoles) of benzoyl chloride is added, and the reaction mixture is stirred at room temperature for 24 hours. Then it is poured onto 150 g of crushed ice, extracted three times with ether, the ether extracts are combined, washed with 2% by weight of aqueous hydrochloric acid, dried over magnesium sulfate, the solvent is distilled off and the residue is recrystallized from methanol. Thus 2.8 g (87 %) of white crystalline product are obtained.
M.p.: 119-120 ^{o}C.
¹H-NMR(CDCl₃): 1.55-1.98 (5H, m, 3CH₂), 2.03 (3H, s, CH₃), 2.07 (3H, s, CH₃), 2.1 (1H, m, CH₂), 2.11 (3H, s, CH₃), 3.80 (1H, t-m, CH), 5.27 (1H, m, C-O), 7.53 (2H, t-m, aromatic pr.), 7.65 (1H, t-m, aromatic pr.), 8.25 (2H, m, aromatic pr.).

### Example 12

### Diisopropyl-[2-(cis-4',6',7'-tri-〈methyl〉-2',3',3'a,8'a-tetra-〈hydro〉-1H-8'-oxacyclopenta[a]indene-5'-yloxy)-ethyl]-amine hydrochloride (I: R₁=R₂=R₃=Me, X=(i-Pr)₂-N-CH₂-CH₂-, A+B=ethylene, n=1)

2.0 g (9.2 mmoles) of the substance prepared according to Example 2 are dissolved in the mixture of 50 ml of dioxane and 20 ml of diethyl ether, and 2.4 g (12.1 mmoles) of 2-di-(isopropylamino)-ethyl chloride hydrochloride and 1.2 g (30 mmoles) of powdered sodium hydroxide are added to the thus obtained solution. The reaction mixture is stirred at 45 ^{o}C for 6 hours, cooled to room temperature, washed with water, dried over magnesium sulfate, and the solvent is distilled off. The residue is dissolved in chloroform, anhydrous hydrogen chloride gas is introduced to it and the solvent is distilled off. The residual substance is recrystallized from a 7 : 3 mixture of acetone and ether. Thus 1.9 g (54 %) of white crystalline product is obtained.
M.p.: 148-150 ^{o}C.
¹H-NMR(CDCl₃): 1.52 (6H, d, J=6Hz, 2CH₃), 1.58 (6H, d, J=6Hz, 2CH₃), 1.6-2.0 (6H, m, 3CH₂), 2.03 (3H, s, CH₃), 2.12 (3H, s, CH₃), 2.19 (3H, s, CH₃), 3.35 (2H, m, 2N-CH), 3.75 (3H, CH₂-N and CH), 4.26 (2H, m, CH₂-O), 5.22 (1H, m, CH-O), 11.68 (1H, s, NH).

### Example 13

### cis-1-[2'-(4'',6'',7''-Tri-〈methyl〉-2'',3'',3''a,8''a-tetra-〈hydro〉-1H-8''-oxacyclopenta[a]indene-5''-yloxy)-ethyl]-piperidine hydrochloride (I: R₁=R₂=R₃=Me, X=2-(piperid-1'-yl)-ethyl, A+B=ethylene, n=1)

To a solution of 2.0 g (9.2 mmoles) of the compound prepared according to Example 2 in 50 ml of anhydrous dimethylformamide 0.96 g of 50% by weight sodium hydride is added, the mixture is stirred for 10 minutes and then 1.87 g (10 mmoles) of 1-[2'(chloro)-eth-1'-yl]-piperidine hydrochloride is added to it. The reaction mixture is stirred at room temperature for 36 hours, poured onto 300 ml of water and extracted five times with ether. The ether extracts are combined, dried over magnesium sulfate, the solvent is distilled off, the residue is dissolved in chloroform and anhydrous hydrogen chloride gas is introduced to it. The chloroform is distilled off and the residue is recrystallized from acetone. Thus 1.8 g (53 %) of white crystalline product is obtained.
M.p.: 201-203 ^{o}C.
¹H-NMR(CDCl₃): 1.4-1.6 (2H, m, 2'-CH₂), 1.6-1.8 (3H, m, 1'-CH₂ and one H of 3-CH), 1.8-2.0 (4H, m, 2CH₂; 4 and 5 CH₂ of the piperidine ring), 2.05 (3H, s, C7-CH₃), 2.08 (1H, m, one H of 3'-CH₂), 2.13 (3H, s, C6-CH₃), 2,21 (3H, s, C4-CH₃), 2.35 (2H, m, CH₂, position 3 of the piperidine ring), 2.93 (2H, m, N-CH₂ piperidine ring), 3.40 (2H, t, J=5Hz, N-CH₂ piperidine ring), 3.73 (3H, m, C3-H and N-CH₂ in the ethyl group), 4.23 (2H, m, O-CH₂), 5.22 (1H, m-t, CH-O), 12.46 (1H, s, NH+).

### Example 14

### cis-4-[2'-(4'',6'',7''-Tri-〈methyl〉-2',3',3'a,8'a-tetra-〈hydro〉-1H-8-oxacyclopenta[a]indene-5''-yloxy)-ethyl]-morpholine hydrochloride (I: R₁=R₂=R₃=Me, X=2-(morpholin-4'-yl)-ethyl, A+B=ethylene, n=1)

To a solution of 2.0 g (9.2 mmoles) of the product prepared according to Example 2 in 50 ml of anhydrous dimethylformamide 0.96 g (20 mmoles) of 50% by weight sodium hydride is added, the mixture is stirred for 15 minutes, and then 1.8 g (9.7 mmoles) of 2-(morpholin-4'-yl)-ethyl chloride hydrochloride is added to it. The reaction mixture is stirred at room temperature for 36 hours, poured onto 300 ml of water and extracted with ether. The ether solution is dried over magnesium sulfate, the solvent is distilled off, the residue is dissolved in chloroform and anhydrous hydrogen chloride gas is introduced to the solution. The chloroform is distilled off, the residue is dissolved in the mixture of 60 ml of carbon tetrachloride and 2 ml of ethyl alcohol under heating and 20 ml of hexane are added to it. The separated crystalline product is filtered off and washed with hexane: Thus 1.6 g (47 %) of white crystalline product is obtained.
M.p.: 175-176 ^{o}C
¹H-NMR(CDCl₃): 1.54 (1H, m, one H of 3'-CH₂), 1.6-1.8 (3H, m, three H of 1'- and 2'-CH₂), 1.8-2.0 (1H, m, one H of 1'- and 2'-CH₂), 2.06 (3H, s, C7-CH₃), 2.08 (1H, m, one H of 3'-CH₂), 2.13 (3H, s, C6-CH₃), 2.21 (3H, s, C4-CH₃), 3.18 (2H, s, CH₂-N), 3.48 (2H, m-t, CH₂-N), 3.70 (1H, bs, C3-H), 3.72 (2H, m-t, CH₂-N), 4.02 (2H, m-d, CH₂O), 4.27 (2H, m, O-CH₂), 4.35 (2H, m-t, CH₂-O), 5.22 (1H, m-t, CH-O), 13.42 (1H, bs, NH+).

### Example 15

### cis-1-[2'-(6'',7''-Di-〈methyl〉-2'',3'',3''a,8''a-tetra-〈hydro〉-1H-8-oxacyclopenta[a]indene-5''-yloxy)-ethyl]-piperidine hydrochloride (I: R₁=R₂=Me, R₃=H, X=2-(piperid-1'-yl)-ethyl, A+B=ethylene, n=1)

To a solution of 2.04 g (10 mmoles) of the compound prepared according to Example 4 in 50 ml of anhydrous dimethylformamide 1.1 g (22.9 mmoles) of 50% by weight sodium hydride is added, and the mixture is stirred for 15 minutes. Then 2.05 g (11 mmoles) of 1-[2'-(chloro)-eth-1'-yl] -piperidine hydrochloride are added to it, the mixture is stirred at room temperature for 24 hours, poured onto 300 ml of water and extracted five times with ether. The ether extracts are combined, dried over magnesium sulfate, the solvent is distilled off, the residue is dissolved in anhydrous ether and hydrochloride salt is formed by introducing hydrogen chloride gas to the solution. The separated crystals are filtered off and washed with anhydrous ether. Thus 1.75 g (50 %) of white crystalline product is obtained. M.p.: 202-203 ^{o}C.
¹H-NMR(CDCl₃): 1.46 (2H, m, CH₂), 1.6-1.95 (8H, m, 4CH₂), 2.06 (3H, s, CH₃), 2.097 (3H, s, CH₃), 2.28 (2H, m, CH₂), 2.86 (2H, m, CH₂-N), 3.42 (2H, m, CH₂-N), 3.67 (2H, m, CH₂-N), 3.79 (2H, m-t, J=7Hz, CH₂-N), 4.45 (2H, m-q, CH₂-O), 5.215 (1H, m, CH-O), 6.55 (1H, s, aromatic pr.), 12.4 (1H, bs, NH).

### Example 16

### cis-[4,6,7-Tri-(methyl)-3a,8a-di-(hydro)-1H-8-oxacyclopenta[a]indene-5-yl] acetate (I: R₁=R₂=R₃=Me, X=Ac, A+B=vinylene, n=1)

To a solution of 4.0 g (18.5 mmoles) of the product prepared according to Example 1 in 20 ml of anhydrous pyridine 3 ml (32 mmoles) of acetic anhydride are added, and the mixture is stirred at room temperature for 6 hours. Then it is poured onto the mixture of 100 g of ice and 3.5 ml of concentrated hydrochloric acid, the separated crystals are filtered off, washed with water and recrystallized from methanol. Thus 0.8 g (17 %) of white crystalline product is obtained.
M.p.: 92-93 ^{o}C.
¹H-NMR(CDCl₃): 1.98 (3H, s, CH₃), 2.02 (3H, s, CH₃), 2.08 (3H, s, CH₃), 2.10 (3H, s, CH₃), 2.45-3.0 (2H, m, CH₂), 4.0 (1H, m, CH), 5.75 (1H, m, CH-O), 5.95 (2H, m, CH=CH).

### Example 17

### cis-[4,6,7-Tri-(methyl)-2,3,3a,8a-tetra-(hydro)-1H-8-oxacyclopenta[a]indene-5-yl] acetate (I: R₁=R₂=R₃=Me, X=Ac, A+B=ethylene, n=1)

A solution of 2.98 g (11.6 moles) of the substance prepared as specified in Example 16 in 150 ml of anhydrous methanol is hydrogenated using 0.5 g of 5% by weight of palladium on charcoal catalyst. When the calculated amount of hydrogen (280 ml, 30 minutes) has been taken up the catalyst is filtered off, the solvent is distilled off and the residue is recrystallized from anhydrous methanol. Thus 2.4 g (80 %) of white crystalline product are obtained.
¹H-NMR(CDCl₃): 1.5-1.95 (5H, m, one H of 2CH₂ and CH₂), 1.99 (3H, s, CH₃), 2.04 (3H, s, CH₃), 2.08 (3H, s, CH₃), 2.10 (1H, m, one H of CH₂), 2.32 (3H, s, CH₃), 3.755 (1H, t+d, J=6Hz and 2HZ, CH), 5.235 (1H, m, CH-O).

### Example 18

### cis-[6-(Methoxy)-2,3,3a,8a-tetra-(hydro)-1H-8-oxacyclopenta[a]indene-5-yl] benzoate (I: R₁=MeO, R₂=R₃=H, X=benzoyl, A+B=ethylene, n=1)

To a solution of 2.1 g of the product prepared according to Example 6 in 10 ml of anhydrous pyridine 1.7 g (12 mmoles) of benzoyl chloride is added, and the mixture is stirred at room temperature for 10 hours. Then it is poured onto the mixture of 30 g of crushed ice and 8 ml of hydrochloric acid, the separated white crystalline product is filtered off, washed with water, dried and recrystallized from ethanol. Thus 2.8 g (90 %) of white crystalline product are obtained. M.p.: 138 ^{o}C.
¹H-NMR(CDCl₃): 1.55 (1H, m, CH₂), 1.65-1.9 (4H, m, 2CH₂), 2.07 (1H, m, dd, CH₂), 3.74 (3H, s, CH₃O), 3.82 (1H, m-t, J=8Hz, CH, 5.3 (1H, m-t, O-CH), 6.42 (1H, s, aromatic pr.), 6.88 (1H, s, aromatic pr.), 7.48 (2H, m, aromatic pr.), 7.6 (1H, m, aromatic pr.), 8.2 (2H, m, aromatic pr.).

### Example 19

### cis-[4,6,7-Tri-(methyl)-2,3,3a,8a-tetra-(hydro)-1H-8-oxacyclopenta[a]indene-5-yl] [3',4',5'-tri-(methoxy)-benzoate] (I: R₁=R₂=R₃=Me, X=3,4,5-tri-(methoxy)-benzoyl, A+B=ethylene, n = 1)

To a solution of 1.9 g (9 mmoles) of the product prepared according to Example 2 in 30 ml of anhydrous pyridine 2.45 g of 3,4,5-tri-(methoxy)-benzoyl chloride are added, and the mixture is starred at room temperature for 15 hours. Then it is poured onto water, the separated precipitate is filtered off, washed with water and recrystallized from methanol. Thus 3.1 g (83.8 %) of the desired product are obtained.
¹H-NMR(CDCl₃): 1.1-1.95 (5H, m, 3CH₂), 2.03 (3H, s, CH₃), 2.08 (3H, s, CH₃), 2.13 (3H, s, CH₃), 2.14 (1H, m, CH₂) 3.8 (1H, m-t, CH), 3,95 (9H, s, 3OCH₃), 5.28 (1H, m, CH-O), 7.5 (s, aromatic pr.).

### Example 20

### Diethyl-[2-(cis-6',7'-di-〈methyl〉-2',3',3'a,8'-tetra-〈hydro〉-1H-8-oxacyclopenta[a]indene-5'-yloxy)-ethyl]-amine hydrochloride (I: R₁=R₂=Me, R₃=H, X=(Et)₂-N-CH₂-CH₂, A+B=ethylene, n=1)

To a solution of 2.5 g (12 moles) of the compound prepared according to Example 4 in 40 ml of anhydrous dioxane 1.3 g (50% by weight oily suspension, 27 moles) of sodium hydride is added in small portions, and the mixture is stirred for 1.5 hours. Then 2.34 g (13.5 moles) of the hydrochloride salt of 2-(diethylamino)-ethyl chloride are added to it, and the reaction mixture is stirred at room temperature for 48 hours. The solvent is distilled off in vacuo, the residue is taken up in 100 ml of ether, washed with water, dried and gaseous hydrogen chloride is introduced to it. The ether is decanted from the separated precipitate and the residue is recrystallized from ethyl methyl ketone. Thus 1.5 g of white crystalline product is obtained.
M.p.: 128-130 ^{o}C.
¹H-NMR(CDCl₃): 1.48 (6H, t, J=7 Hz, 2CH₃), 1.5-2.0 (5H, m, CH₂), 2.05 (1H, m, CH₂), 2.07 (3H, s, CH₃), 2.10 (3H, s, CH₃), 3.29 (4H, m, 2 N-CH₂), 3,80 (1H, m-t, J=7 Hz and 1.5 Hz, CH), 4.42 (2H, m, O-CH₂), 5.22 (1H, m, J=7 Hz and 1.5 Hz, CH), 6.52 (1H, s, aromatic H), 12.8 (1H, br s., NH⁺).

### Example 21

### cis-4,6-Di-(methoxy)-3a,8a-di-(hydro)-3H-8-oxacyclopenta[a]indene-5-ol (I: R₁=R₃=MeO, R₂=X=H, A+B=vinylene, n=1)

To a solution of 5.0 g (29 mmoles) of 2,6-di-(methoxy)-hydroquinone (II: R₁=R₃=MeO, R₂=R₄=H) and 3.5 g (29 mmoles) of cis-cyclopent-4-ene-1,3-diol (III: n=1) in 30 ml of anhydrous toluene 0.5 g of p-toluenesulfonic acid is added, and the reaction mixture is stirred at a temperature of 70 ^{o}C for 3 hours. Then it is cooled, diluted with 200 ml of ethyl acetate, washed with water and saturated sodium chloride solution, dried and the solvent is distilled off. The residue is purified by column chromatography (Kieselgel G., hexane:acetone 10:1) and recrystallized from hexane. Thus 1.29 g (21 %) of light yellow crystalline product is obtained.
M.p.: 140 ^{o}C.
¹H-NMR: 2.67 and 2.80 (2H, m, CH₂), 3.80 (3H, s, OCH₃), 3.96 (3H, s, OCH₃), 4.16 (1H, dd, J=8 and 2.5 Hz, CH), 5.11 (1H, s, OH), 5.78 (1H, dd, J=8 and 2.5Hz, CH-O), 5.80 (1H, m, CH=), 6.00 (1H, m, CH=), 6.16 (1H, s, aromatic pr.).

### Example 22

### cis-6-(Propyloxy)-3a,8a-di-(hydro)-3H-8-oxacyclopenta[a]indene-5-ol (I: R₁=PrO, R₂=R₃=X=H, A+B=vinylene, n=1)

To a suspension of 5.0 g (22 mmoles) of 2,5-di-(propoxy)-hydroquinone (II: R₁=R₄=PrO, R₂=R₃=H) and 2.4 g (24 mmoles) of cis-cyclopent-4-ene-1,3-diol (III: n=1) in 50 ml of anhydrous toluene 0.2 g of D-camphor-10-sulfonic acid is added, and the mixture is stirred under nitrogen atmosphere at a temperature of 65 ^{o}C for 30 hours. The separated precipitate is filtered off, the filtrate is evaporated and the residue is purified by column chromatography. Thus 0.7 g (28 %) of white crystalline substance is obtained.
M.p.: 69 ^{o}C.
¹H-NMR(CDCl₃): 1.02 (3H, t, J=6.5Hz, CH₃), 1.8 (2H, sexst., J=6.5 Hz, CH₂), 2.54 and 2.86 (2H, m, CH₂), 3.91 and 3.94 (2H, m, CH₂-O), 4.00 (1H, m, CH), 5.28 (1H, s, OH), 5.78 (1H, m, CH-O), 5.82 (1H, m, CH=), 6.00 (1H, m, CH=), 6.37 (1H, s, aromatic pr.), 6.73 (1H, s, aromatic pr.).

### Example 23

### cis-1,3,4-Tri-(methyl)-5a,8,9,9a-tetra-(hydro)-dibenzofurane-2-ol (I: R₁=R₂=R₃=Me, X=H, A+B=vinylene, n=2)

To a solution of 5.0 g (33 mmoles) of trimethylhydroquinone (II: R₁=R₂=R₃=Me, R₄=H) and 5.0 g (44 mmoles) of cis-cyclohex-2-en-1,4-diol (III: n=2) in 100 ml of anhydrous toluene 1.0 g of D-camphor-10-sulfonic acid is added, and the mixture is stirred at a temperature of 70 ^{o}C for 40 hours. Then it is cooled, diluted with 100 ml of ether, washed twice with water and once with saturated sodium chloride solution, dried and the solvent is distilled off. The residue is purified by column chromatography (Kieselgel G, hexane: ether 1:1) and recrystallized from hexane. Thus 2.0 g (25.74 %) of white crystalline product are obtained.
M.p.: 122-123°C.
¹H-NMR(CDCl₃): 1.34 (1H, m, one H of CH₂), 1.8-2.6 (3H, m, CH₂ and one H of CH₂), 2.11 (6H, s, 2CH₃), 2.18 (3H, s, CH₃), 3.13 (1H, m, CH), 4.26 (1H, s, OH), 4.74 (1H, dd, J=7 and 2.5 Hz, CH-O), 6.12 (1H, m, CH=), 6.14 (1H, m, CH=).

### Example 24

### cis-[4,6,7-Tri-(methyl)-3a,8a-di-(hydro)-1H-8-oxacyclopenta[a]indene-5-yl] [4'-(nitro)-benzoate] (I: R₁=R₂=R₃=Me, X=4-(nitro)-benzoyl, A+B=vinylene, n=1)

To a solution of 5.0 g (23 mmoles) of the substance prepared according to Example 1 in 50 ml of anhydrous pyridine 4.5 g (24 mmoles) of 4-(nitro)-benzoyl chloride are added, and the reaction mixture is stirred at room temperature for 10 hours. Then it is added to a mixture of 2N hydrochloric acid and crushed ice, the separated crystals are filtered off, washed with water and recrystallized from ethanol. The separated crystalline product is recrystallized four times from acetone. Thus 1.0 g (11.8 %) of yellow crystalline product are obtained.
M.p.: 170-173 ^{o}C.
¹H-NMR(CDCl₃): 2.02 (3H, s, CH₃), 2.08 (3H, s, CH₃), 2.12 (3H, s, CH₃), 2.55 (1H, m, one H of CH₂), 2.93 (1H, m, one H of CH₂), 4.13 (1H, t-m, J=4Hz, CH), 5.86 (1H, d-m, J=4Hz, CH-O).

### Example 25

### cis-[6,7-Di-(methyl)-3a,8a-di-(hydro)-3H-8-oxacyclopenta[a]indene-5-yl] benzoate (I: R₁=R₂=Me, R₃=H, X=benzoyl, A+B=vinylene, n=1)

To a solution of 2.65 g (13 moles) of the substance prepared according to Example 3 in 15 ml of anhydrous pyridine 1.92 g (1.6 ml, 14 moles) of benzoyl chloride is added, and the mixture is stirred at room temperature for 15 hours. Then it is poured onto a mixture of 50 g of ice and 5 ml of concentrated hydrochloric acid, the separated solid substance is filtered off and recrystallized from ethanol. Thus 2.2 g (55 %) of white crystalline product are obtained.
¹H-NMR(CDCl₃): 2.05 (3H, s,CH₃), 2.15 (3H, s, CH₃), 2.56 (1H, m, one H of CH₂), 2.9 (1H, m dd, one H of CH₂), 4.08 (1H, m-t, CH), 5.8-5.9 (2H, m, CH and CH=), 6.8 (1H, s, aromatic pr.), 7.5 (2H, m, aromatic pr.), 7.64 (1H, m, aromatic pr.), 8.22 (2H, m, aromatic pr.).

### Example 26

### cis-3-(Ethoxy)-5a,8,9,9a-tetra-(hydro)-dibenzofurane-2-ol (I: R₁=R₃=X=H, R₁=EtO, A+B=vinylene, n=2)

To a solution of 20.7 g (0.1 moles) of 2-(ethoxy)-hydroquinone and 0.8 g of p-toluenesulfonic acid in 200 ml of toluene 12.9 g (0.11 moles) of cis-cyclohex-2-en-1,4-diol (III: n=2) are added, and the mixture is stirred at a temperature of 70 ^{o}C, under nitrogen atmosphere for 24 hours. Then it is filtered, the filtrate is evaporated, the residue is purified by column chromatography and recrystallized from hexane. Thus 3.3 g (14.1 %) of the desired product are obtained.
M.p.: 92-94 ^{o}C.
¹H-NMR(CDCl₃): 1.41 (3H, t, J=7Hz, CH₃), 1.55 (1H, m, CH₂), 1.9 (2H, m, CH₂), 2.1 (1H, m, CH₂), 3.31 (1H, m, CH), 4.04 (2H, q, J=7Hz, CH₂), 4.95 (1H, m, CH), 5.3 (1H, s, OH), 5.92 (1H, m, CH=), 6.11 (1H, m, CH=), 6.4 (1H, s, arom.H), 6.77 (1H, s, arom. H).

### Example 27

### cis-1,3,4-Tri-(methyl)-5a,6,7,8,9,9a-hexa-(hydro)-dibenzofurane-2-ol (I: R₁=R₂=R₃=Me, A+B=ethylene, X=H, n=2)

To a solution of 3.0 g (19.7 mmoles) of trimethylhydroquinone (II: R₁=R₂=R₃=Me) and 3.16 g (39.5 mmoles) of cyclohexa-1,3-diene (IV: n=2) in 70 ml of toluene 0.6 g (3.4 mmoles) of p-toluenesulfonic acid is added, and the mixture is kept at 100 ^{o}C for 15 minutes. Then it is cooled, diluted with 150 ml of ether, extracted with 10% by weight of sodium hydrogencarbonate solution and water, dried, the solvent is distilled off and the residue is recrystallized from a 7 : 3 mixture of acetone and hexane. Thus 3.4 g (43 %) of white crystalline product are obtained.
M.p.: 115-118 ^{o}C.
¹H-NMR (CDCl₃): 1.1 and 1.22 (2H, m, CH₂), 1.65 (4H, m, 2CH₂), 1,92 (1H, m, CH), 2.13 (3H, s, CH₃), 2.14 (3H, s, CH₃), 2.153 (3H, s, CH₃), 2.59 (1H, m, CH), 2.96 (1H, m, CH), 4.2 (1H, s, OH), 4.46 (1H, m, CH-O).

### Example 28

### cis-1,3-Di-(methoxy)-5a,6,7,8,9,9a-hexa-(hydro)-dibenzofurane-2-ol (I: R₁=R₃=MeO, R₂=H, A+B=ethylene, X=H, n=2)

To a solution of 1.7 g (10 mmoles) of 2,6-di-(methoxy)-hydroquinone (III: R₁=R₃=MeO, R₂=H) and 1.5 g (19 mmoles) of cyclohexa-1,3-diene (IV : n=2) in 30 ml of toluene 1.9 g of p-toluenesulfonic acid is added, and the mixture is stirred first at 70 ^{o}C for 15 hours and then at 100 ^{o}C for 5 hours. The reaction mixture is then cooled, diluted with 30 ml of ether, washed with water and saturated sodium chloride solution, dried and the solvent is distilled off. The residue is purified by column chromatography. Thus 0.9 g (36 %) of white crystalline product is obtained.
M.p.: 97-98 ^{o}C.
¹H-NMR (CDCl₃): 1.2-2.1 (8H, m, CH₂), 3.1 (1H, m, CH), 3.78 (3H, s, OCH₃), 3.95 (3H, s, OCH₃), 4,55 (1H, m, CH-O), 5.2 (1H, bs, OH), 6.2 (1H, s, aromatic H).

### Example 29

### cis-1,2-Di-(methyl)-4b,6,7,8,9,9a-hexa-(hydro)-5H-10-oxabenzo[a]azulene-3-ol (I: R₁=R₂=Me, R₃=H, A+B=ethylene, X=H, n=3)

To a solution of 2.0 g (14 mmoles) of 2,3-di-(methyl)-hydroquinone (II: R₁=R₂=Me, R₃=R₄=H) and 1.63 g (17 mmoles) of cyclohepta-1,3-diene (IV : n=3) in 50 ml of anhydrous toluene 0.2 g of p-toluenesulfonic acid is added, and the mixture is stirred at a temperature between 70 ^{o}C and 80 ^{o}C for 18 hours. Then it is cooled, diluted with 100 ml of ether, washed with water and saturated sodium chloride solution, the solvent is distilled off and the residue is purified by column chromatography. Thus 1.2 g (37%) of white crystalline product is obtained.
M.p.: 105-107 ^{o}C.
¹H-NMR (CDCl₃): 1.3-2.0 (8H, m, 4CH₂), 2.12 (6H, s, 2CH₃), 2.4 (2H, m, CH₂), 2.85 (1H, m, CH), 4.2 (1H, m, OH), 4.8 (1H, m, CH-O), 6.4 (1H, s, aromatic H).

## Claims

1. Oxaindene derivatives of general formula 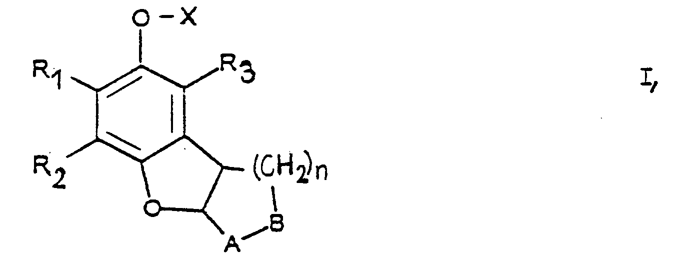 wherein
R₁ represents a C₁₋₄-alkyl, C₁₋₄-alkoxy or C₃₋₆-cycloalkyl group,
R₂ stands for hydrogen or a C₁₋₄-alkyl or C₃₋₆-cycloalkyl group,
R₃ represents hydrogen or a C₁₋₄-alkyl, C₁₋₄-alkoxy or benzyloxy group,
X means hydrogen, an aliphatic C₁₋₄-acyl or benzoyl or naphthoyl group, optionally substituted by 1 or more nitro or C₁₋₄-alkoxy group(s) or a group of formula 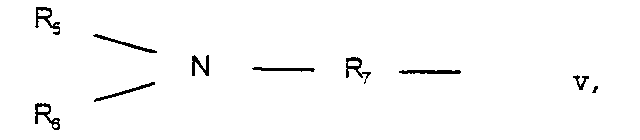 in which latter
R₇ stands for a C₁₋₄-alkylene group
and
R₅ and R₆ each represents a C₁₋₄-alkyl group
or
R₅ and R₆ together with the nitrogen atom to which they are attached form a piperidyl or morpholinyl group,
A and B together form an ethylene or a vinylene group
and
n is 1, 2 or 3,
and their stereoisomers and their mixtures as well as acid addition salts of these compounds.

2. Oxaindene derivatives according to claim 1, characterized by that the alkyl group(s) which may be represented by R₁ , R₂ , R₃ , R₅ and/or R₆ is/are such having from 1 to 3, particularly 1 or 2, carbon atom(s).

3. Oxaindene derivatives according to claim 1 or 2, characterized by that the alkoxy group(s) which may be represented by R₁ and/or R₃ and/or by which the benzoyl or naphthoyl group which may be represented by X may be substituted is/are such having from 1 to 3, particularly 1 or 2, carbon atom(s).

4. Oxaindene derivatives according to claims 1 to 3, characterized by that the cycloalkyl group(s) which may be represented by R₁ and/or R₂ is/are such having 5 or 6 carbon atoms.

5. Oxaindene derivatives according to claims 1 to 4, characterized by that the alkylene group which may be represented by R₇ is such having from 1 to 3, particularly 2 or 3, carbon atom(s).

6. Oxaindene derivatives according to claims 1 to 5, characterized by that the aliphatic acyl group which may be represented by X is such having from 1 to 3, particularly 2 or 3, carbon atom(s).

7. Oxaindene derivatives according to claims 1 to 6, characterized by that
R₁ and R₃ independently represent hydrogen or C₁₋₄-alkyl or C₁₋₄-alkoxy groups,
R₂ stands for hydrogen or a C₁₋₄-alkyl group,
X represents hydrogen,
A and B together form a vinylene group
and
n is 1 or 2.

8. Oxaindene derivatives according to claims 1 to 6, characterized by that
R₁ and R₃ independently represent hydrogen or C₁₋₄-alkyl or C₁₋₄-alkoxy groups,
R₂ stands for hydrogen or a C₁₋₄-alkyl group,
X represents hydrogen,
A and B together form an ethylene group
and
n is 1 or 3.

9. cis-4,6,7-Tri-(methyl)-2,3,3a,8a-tetra-(hydro)-1H-8-oxacyclopenta[a]indene-5-ol, 1,2,4-tri-(methyl)-4b,6,7,8,9,9a-hexa-(hydro)-5H-10-oxabenzo[a]azulene-3-ol and cis-4,6,7-tri-(methyl)-3a,8a-di-(hydro)-3H-8-oxacyclopenta-[a]indene-5-ol.

10. Process for preparing the oxaindene derivatives according to claims 1 to 9, characterized by that
a) for preparing oxaindene derivatives of general formula I, in which A and B together form a vinylene group and R₁ , R₂ , R₃ , X and n are as defined in claims 1 to 8, reacting hydroquinone derivatives of general formula 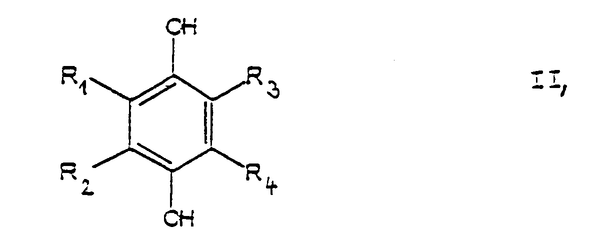 wherein R₁ , R₂ and R₃ are as defined in claims 1 to 4, 7 or 8 and R₄ represents hydrogen or a C₁₋₄-alkoxy group, with cycloalkene derivatives of general formula 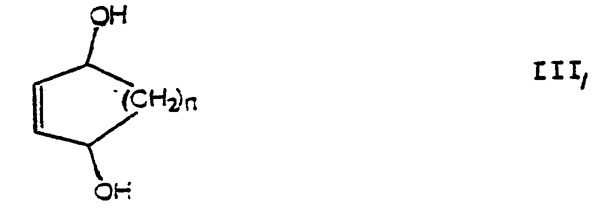 wherein n is as defined in claim 1, 7 or 8, in solvents in the presence of catalytic amounts of strong acids, and, in a manner known per se, optionally subjecting thus obtained oxaindene derivatives of general formula I, wherein X represents hydrogen, to acylation or dialkylaminoalkylation
or
b) for preparing oxaindene derivatives of general formula I, in which A and B together form an ethylene group,
b₁) in a manner known per se subjecting oxaindene derivatives of general formula I, wherein A and B together form a vinylene group, X stands for hydrogen and R₁ , R₂ , R₃ and n are as defined in claims 1 to 4, 7 or 8, to catalytic hydrogenation,
or
b₂) reacting hydroquinone derivatives of general formula II, wherein R₁ , R₂ and R₃ are as defined in claims 1 to 4, 7 or 8 and R₄ represents hydrogen, with cycloalkadiene derivatives of general formula 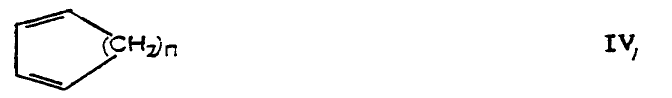 wherein n is as defined in claims 1, 7 or 8, in solvents in the presence of catalytic amounts of strong acids,
and, in a manner known per se, optionally subjecting thus obtained oxaindene derivatives of general formula I, wherein X represents hydrogen, to acylation or dialkylaminoalkylation,
and, in a manner known per se, if desired, converting oxaindene derivatives of general formula I thus obtained into acid addition salts thereof or, if desired, liberating free oxaindene derivative bases of general formula I from salts thereof and, if desired, converting the free oxaindene derivative bases into other acid addition salts and/or, if desired, separating the stereoisomers.

11. Process according to claim 10a), characterized by using for the reaction of the hydroquinone derivatives of general formula II with the cycloalkene derivatives of general formula III as solvent(s) [an] aprotic solvent(s), particularly 1 or more aromatic hydrocarbon(s).

12. Process according to claim 10a) or b₂) or 11, characterized by using for the reaction of the hydroquinone derivatives of general formula II with the cycloalkene derivatives of general III or with the cycloalkadiene derivatives of general formula IV as strong acid p-toluenesulfonic acid, camphor-10-sulfonic acid or benzenesulfonic acid.

13. Process according to claim 10a), 11 or 12, characterized by carrying out the reaction of the hydroquinone derivatives of general formula II with the cycloalkene derivatives of general formula III at elevated temperatures, particularly of from 60 to 110°C.

14. Process according to claim 10b₁), characterized by using as solvent(s) for the catalytic hydrogenation of the oxaindene derivatives of general formula I, wherein A and B together form a vinylene group, X stands for hydrogen and R₁ , R₂ , R₃ and n are as defined in claims 1 to 4, 7 or 8, 1 or more polar solvent(s).

15. Process according to claim 10b₁) or 14, characterized by using as catalyst for the catalytic hydrogenation of the oxaindene derivatives of general formula I, wherein A and B together form a vinylene group, X stands for hydrogen and R₁ , R₂ , R₃ and n are as defined in claims 1 to 4, 7 or 8, palladium.

16. Process according to claim 10b₁), 14 or 15, characterized by carrying out the catalytic hydrogenation of the oxaindene derivatives of general formula I, wherein A and B together form a vinylene group, X stands for hydrogen and R₁ , R₂ , R₃ and n are as defined in claims 1 to 4, 7 or 8 at room temperature.

17. Process according to claim 10b₂) or 12, characterized by carrying out the reaction of the hydroquinone derivatives of general formula II with the cycloalkadiene derivatives of general formula IV in 1 or more anhydrous aprotic solvent(s).

18. Process according to claim 10b₂), 12 or 17, characterized by carrying out the reaction of the hydroquinone derivatives of general formula II with the cycloalkadiene derivatives of general formula IV at elevated temperatures, particularly of from 30 to 90°C.

19. Medicaments, characterized by that they contain as active principle(s) at least one oxaindene derivative according to claims 1 to 9, suitably in admixture with at least one inert solid and/or liquid pharmaceutical carrier and/or diluent and/or further auxiliary excipient.

20. Use of the oxaindene derivatives according to claims 1 to 9 for the preparation of medicaments, particularly such having activities against diseases of allergy, diseases of the joints, asthma, diseases of the gastro-intestinal tract including ulcers and skin disorders.
